# EUROPEAN PATENT APPLICATION

(11) **EP 1 273 656 A1**
(43) Date of publication of application: **08.01.2003**
(21) Application number: 01401795.8
(22) Date of filing: 05.07.2001
(51) Int. Cl.: C12N 15/11, A61K 31/7088, A61P 35/00

(54) **Use of inhibitors of expression or activity of p8/com1 for treating tumours**

(71) Applicant: INSTITUT NATIONAL DE LA SANTE ET DE LA RECHERCHE MEDICALE (INSERM), 75013 Paris (FR)
(72) Inventor: Vasseur, Sophie, 13010 Marseille (FR); Iovanna, Juan, 3 traverse de la Roué, 13010 Marseille (FR); Dagorn, Jean-Charles, 13009 Marseille (FR)
(74) Representative: Goulard, Sophie

(57) **Abstract**

The invention relates to the use of inhibitors of expression or activity of p8/com1 for treating tumours.

## Description

The invention relates to the use of inhibitors of expression or activity of p8/com1 for treating tumours.

Recent advances in molecular genetics helped unravelling some of the molecular mechanisms involved in the occurrence and progression of cancer, and several genes involved in induction or suppression of tumour progression and metastasis have been described.

Cancer progression occurs in several steps. During transformation, some cancer cells are positively selected within the tumour on the basis of their growth capacity, low response to apoptotic signals and ability to escape the immunological survey of the host. After leaving the primary tumour, transformed cells migrate through the body. Yet, metastases will not develop in all tissues. Capacity for invading the target organ is a first limitation. But once within the organ, metastasis will develop only if transformed cells can cope with their new microenvironment. Being different from the original environment to which the phenotype of transformed cells is adapted, it will induce some stress to the cells. Therefore, it has been hypothesized that stress-associated genes may facilitate tumour progression and metastasis formation by helping the cells to adapt to the host micro-environment. Supporting that hypothesis, several stress-associated genes were found overexpressed in tumours (STORM *et al*., Ann. Surg. Oncol., 3, 570-573, 1996; JAMORA *et al*., Proc. Natl. Acad. Sci. USA, 93, 7690-7694, 1996) and their expression level correlated with aggressiveness. However, the understanding of the underlying mechanisms remains limited and the actual involvement of cellular stress-response mechanisms in tumour spreading has not been demonstrated until now.

In the past, the inventors have identified a new gene, called p8, activated in pancreatic acinar cells during the acute phase of pancreatitis. This gene (GenBank AF069073) encodes a 82 amino acid peptide related to HMG-I/Y DNA-binding proteins (ENCINAR *et al*., J. Biol. Chem., 276, 2742-2751, 2001). It was shown to be also involved in pancreatic development and regeneration, and to promote cellular growth (MALLO *et al.,* J. Biol. Chem., 272, 32360-32369, 1997).

Further experiments have shown that p8 activation is not restricted to pancreatic cells, and *in vitro* studies showed that a variety of cell lines exhibited transient p8 mRNA expression in response to several stress agents (MALLO *et al*., J. Biol. Chem., 272, 32360-32369, 1997; VASSEUR *et al.,* Eur. J. Biochem., 259, 670-675, 1999).

Later, REE *et al*. (Cancer Res., 59, 4675-4680, 1999) described a factor, called com1 (for: "candidate of metastasis 1") that is up-regulated in breast cancer metastatic cells. This factor turned out to be identical to human p8. These authors hypothesized that the expression of this factor could mediate the growth of tumour cells after metastatic establishment in a secondary organ. On the other hand, the same team (BRATLAND *et al*., Cancer Res. 60, 5578-5583, 2000) recently suggested that rather than promoting growth, com1 may participate in the regulation of cellular growth inhibition when recruited by inhibitory signals.

The inventors have now found that disruption of the p8 gene is sufficient to prevent tumourogenicity of E1A/ras transformed MEFs (mouse embryo fibroblasts). Thus, p8 represents a new drug-targetable gene to treat tumour progression and metastasis development.

The invention relates to the use of an inhibitor of the expression or of the activity of p8/com1 for preparing an antitumoral medicament.

Several methods for the targeted inhibition of the expression of a gene are known in themselves (for review cf. for instance GEWIRTZ *et al*., Blood, 92, 712-736, 1998; JEN and GEWIRTZ, Stem Cells, 18, 307-319, 2000).

Suitable inhibitors of the expression of p8/com1 include for instance:
- molecules down-regulating the transcription of the p8 gene, such as triple-helix-forming molecules capable of sequence-specific binding with double-helical DNA in the p8 promoter; triple-helix-forming molecules have been used for inhibiting transcription of various genes (GIOVANNANGELI and HELENE, Curr. Opin. Mol. Ther., 2(3): 288-296, 2000);
- molecules decreasing the stability of the p8 mRNA and/or down-regulating its translation, such as antisense molecules specifically binding with p8/com1 mRNA, or ribozymes directed to cleave the p8 mRNA.

Appropriately targeted triple-helix-forming molecules, antisense molecules or ribozymes can easily be designed from the known sequence of the gene p8/com1. Typically, said molecules include oligonucleotides and oligonucleotide analogues.

One can use natural DNA or RNA oligonucleotides; however, in many instances, it is preferred to use oligonucleotides chemically modified in order to increase their stability, their nuclease resistance, their affinity for their target sequence, and/or their activity. A number of such modifications have been described in the literature, (for review, cf. IYER *et al*., Curr. Opin. Mol. Ther., 1, 344-358, 1999).

Non-limitative examples of chemically modified oligonucleotides include for instance phosphorothioate oligonucleotides, that are widely used for *in vivo* antisense applications, or phosphoramidate oligonucleotides, that can be used either as triple-helix-forming molecules and as antisense molecules (FARIA *et al.,* Proc. Natl. Acad. Sci. USA, 97, 3862-3867, 2000; FARIA *et al.,* Nature Biotech., 19, 40-44, 2001).

Suitable oligonucleotide analogues include for instance peptide nucleic (PNAs) acids (NIELSEN *et al.,* Anticancer Drug Des., 8, 53-63, 1993).

These inhibitors of p8/com1 activity may be administered by a variety of methods; many of them are indicated for instance in the publications of GEWIRTZ *et al.* and JEN and GEWIRTZ cited above. The choice of an appropriate method depends *inter alia* on the chemical nature of the molecule, on the target organ or tissue, and/or on whether a short-time inhibition or a long-lasting inhibition is wanted.

By way of example, natural RNA molecules can be expressed inside the target cells from an expression vector transfected into said cells.

Natural DNA or RNA oligonucleotides, as well as chemically modified oligonucleotides, or oligonucleotide analogues can also be delivered to cells externally. For instance, they can be administered by themselves, or packaged into liposomes, or conjugated to carrier proteins or peptides, such as cell-penetrating peptides (LIDGREN *et al.,* TiPS, 21, 99-102, 2000).

The invention also comprises pharmaceutical compositions comprising an inhibitor of p8/com1 expression or activity, as defined above, associated with suitable carriers or excipient(s). The invention may also comprise one or several other anti-tumoral molecules.

Pharmaceutical compositions of the invention can be used systemically or locally. A preferred route of systemic administration is the parenteral route, including for instance intramuscular, subcutaneous, intravenous, or intraperitoneal injections. A preferred route of local administration is intra-tumoral injections.

The oral route can also be used, provided that the medicament is in a form suitable for oral administration, able to protect the active principle from the gastric and intestinal enzymes.

Inhibitors of p8/com can be used in the treatment of many types of tumours, for instance tumours of breast, pancreas, prostate, etc.

The present invention will be further illustrated by the additional description which follows, which refers to examples illustrating the effects of disruption of the p8 gene on the tumourogenicity of transformed cells. It should be understood however that these examples are given only by way of illustration of the invention and do not constitute in any way a limitation thereof.

### EXAMPLE 1: CONSTRUCTION OF KNOCKOUT MICE DEFICIENT IN P8 EXPRESSION

To investigate the function of p8 in cancer progression, a null allele was generated in 129/Sv embryonic stem (ES) cells by replacing exon 2 of the p8 gene, which encodes 60% of the coding sequence, with a neomycin-resistance cassette, using a conventional targeting vector (pBS-TK-NeoLox).

Germline transmission was obtained after injection of these ES cells into C57BL/6 blastocysts. Then, mice heterozygous for the targeted disruption were intercrossed to produce homozygous offsprings. These crosses provided litters of normal size, with living, apparently normal p8^{-/-} offsprings occurring with a frequency consistent with Mendelian inheritance.

p8 deficiency was verified by RT-PCR and/or Southern blot analysis and by immunostaining of the pancreas with acute pancreatitis using a p8 polyclonal antibody (data not shown).

### EXAMPLE 2: TRANSFORMATION OF PRIMARY EMBRYO FIBROBLASTS FROM P8^{+/+} AND P8^{-/-} MICE.

To explore the function of p8/com1 in cancer progression, embryonic fibroblasts obtained from knockout p8^{-/-} mice and their p8^{+/+} counterpart were used. These fibroblasts were transformed and their growth properties *in vitro* and *in vivo* were compared.

### Preparation of p8^{+/+} and p8^{-/-} MEFs

Primary embryo fibroblasts (MEFs) were isolated from 14.5 day-old mouse embryos following standard protocols (HARVEY *et al*., Oncogene, 8, 2457-2467, 1993).

Cells were grown in Dulbecco's modified Eagle's medium supplemented with 10% foetal calf serum. Expression of p8 mRNA in p8^{+/+} and p8^{-/-} mouse embryo fibroblasts (MEF) was evaluated by RT-PCR using specific primers.

RNA was extracted using Trizol (Life Technologies) procedures. Total RNA (1 ug) was analysed by RT-PCR with the SuperScriptTM One-step RT-PCR System with Platinum Taq kit (Life Technologies). The RT-PCR was performed using different numbers of cycles to verify that PCR was inside the linear range. The mRNA coding for p8 was specifically amplified with sense: (5'GGAGAGAGCAGACTAGGCATA3'): SEQ ID NO: 1 and antisense: (5'GTTGCTGCCACCCAAGGGCAT3'): SEQ ID NO: 2 primers, in positions 16 and 581 of the cDNA, respectively. As a positive control, the transcript coding for the ribosomal protein RL3 was specifically amplified for 19 cycles with sense: (5'GAAAGAAGTCGTGGAGGCTG3'): SEQ ID NO: 3 and antisense: (5'ATCTCATCCTGCCCAAACAC3'): SEQ ID NO: 4 primers, in positions 216 and 637 of the cDNA, respectively.

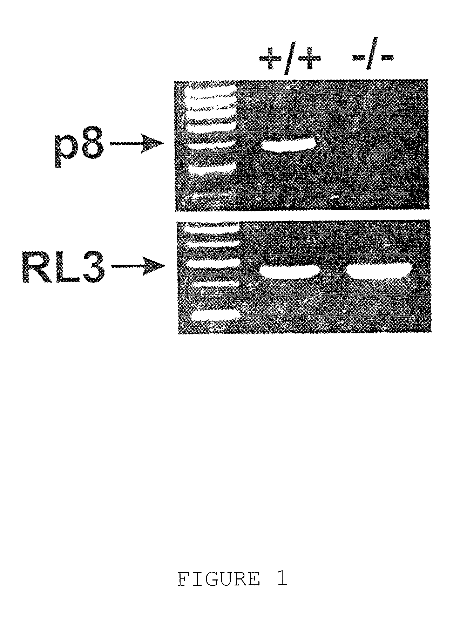

The results are represented in Figure 1. These results show that p8 mRNA is expressed only in p8^{+/+} MEFs.

### Transformation of the MEFs

### Methods

Oncogenic ras transforms most immortal rodent cells to a tumorigenic state, whereas transformation of primary cells requires either a cooperating oncogene or the inactivation of a tumour suppressor. The adenovirus E1A oncogene cooperates with ras to transform primary rodent fibroblasts (RULEY *et al*., Cancer Cells, 2, 258-268, 1990) and abrogates ras-induced senescence (SERRANO *et al.,* Cell, 88, 593-602, 1997). Therefore, p8^{+/+} and p8^{-/-} MEFs were transduced with the pBabe-ras^{V12}/E1A retroviral vector which expresses both the ras^{V12} mutated protein and the E1A oncogene to obtain transformed fibroblasts with a defined p8 status.

pBabe-ras^{V12}/E1A and pBabe plasmids are described in SERRANO *et al.* cited above.

Bosc 23 ecotropic packaging cells (10⁶) were plated in a 6-well plate, incubated for 24 hr, and then transfected with PEI with 5 µg of retroviral plasmid. After 48 hr, the medium containing the virus was filtered (0.45 µm filter, Millipore) to obtain the "first supernatant".

Target MEFs were plated at 2 x 10⁵ cells per 35 mm dish and incubated overnight. For infections, the culture medium was replaced by an appropriate mix of the first supernatant and culture medium (V/V), supplemented with 4 µg/ml polybrene (Sigma), and cells were incubated at 37°C. Forty eight hours later infected cell populations were selected by culture in the presence of puromycin (0.7 µg/ml) for 5 days to eliminate uninfected cells.

In all experiment, cells infected with the empty pBabe vector were used as negative control.

### Results

Transformation of p8^{+/+} and p8^{-/-} MEFs by the pBabe-ras^{V12}/E1A retroviral vector was evaluated by examining changes in their morphological aspect, by quantifying expression of the RAS protein by western blot, and by monitoring cell proliferation.

### Morphological aspect

Transduction of MEFs with the pBabe-ras^{V12}/EIA retroviral vector induced important morphological changes typical of cell transformation (i.e. cells lost their flat and enlarged cytoplasm and became refractile with thin projections) while no change in morphology was observed in cells transduced with the pBabe retroviral vector.

### Western-blot analysis

One hundred µg of total cell protein was separated with standard procedures on 12.5% SDS-PAGE using the Mini Protean System (Bio-Rad) and transferred to nitrocellulose membrane (Sigma). The intracellular level of RAS was estimated by Western blot using the H-ras (C-20) polyclonal antibody (1:200) purchased from Santa Cruz Biotechnology, Inc.

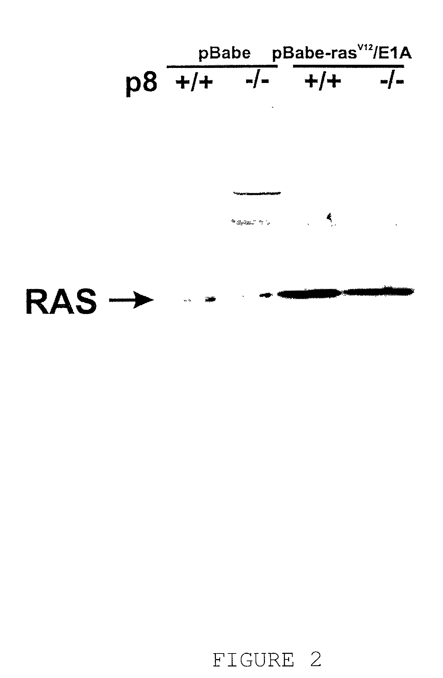

Figure 2 represents the results of immunoblot analysis of p8^{+/+} and p8^{-/-} MEFs transduced with pBabe (control) or pBabe-ras^{V12}/E1A (transformed) retroviruses. Error bars represent the SEM. Both transformed p8^{+/+} and p8^{-/-} MEFs expressed high levels of RAS compared to empty vector transduced control cells.

### Growth curves

One hundred thousand cells per well were plated in a series of 35 mm culture dishes. The cell number was estimated daily in triplicate (from 1 to 7 days) in a haemocytometer, and each curve was established at least two times.

Figure 3 represents growth curves corresponding to p8^{+/+} and p8^{-/-} MEFs transduced with pBabe or pBabe-ras^{V12}/E1A retroviruses.

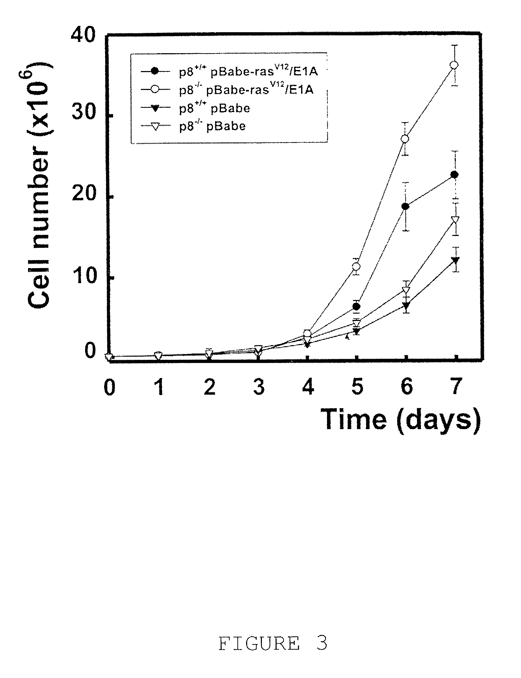

Legend of Figure 3:
―●― : p8⁺/⁺ pBabe-ras^{V12}/E1A
―○― : p8⁻/⁻ pBabe-ras^{V12}/E1A
―▼―: p8⁺/⁺ pBabe
―∇― : p8⁻/⁻ pBabe

Transformed fibroblasts grew 2-3 fold faster than control MEFs. It is noteworthy that lack of p8 expression did not affect cell growth. On the contrary, p8^{-/-} and transformed p8^{-/-} MEFs grew more rapidly than p8^{+/+} and transformed p8^{+/+} fibroblasts, respectively.

Based on these results it can be assumed that p8^{+/+} and p8^{-/-} MEFs were transformed by the pBabe-ras^{V12}/E1A retroviral vector. The similar features acquired by p8^{+/+} and p8⁻/⁻MEFs upon transformation by ras^{V12}/E1A suggested that p8 deficiency did not interfere with MEF transformation.

### EXAMPLE 3: TUMOUROGENICITY PROPERTIES OF TRANSFORMED P8^{+/+} AND P8^{-/-} MEFS.

The tumourogenicity properties of transformed p8^{+/+} and p8^{-/-} MEFs were assessed by checking their ability to grow *in vitro* and to form tumours in immunocompromised mice.

### Colony growth assays

To determine the ability of different cell lines to grow in soft-agar, 5 x 10⁴ cells were resuspended in 0.3% agar in DMEM plus 10% FCS and overlaid on 0.6% agar in the same medium in 12-well plates. Experiments were performed in triplicate and repeated two (pBabe-transduced cells) or three times (pBabe-ras^{V12}/E1A-transduced cells).

In this assay, transformed p8^{+/+} MEFs formed colonies at high frequency, as expected, but p8^{-/-} transformed MEFs, like control p8^{+/+} or p8^{-/-} MEFs transduced with the pBabe control vector, were unable to form colonies.

### In vivo tumour growth, spreading assays and histological analysis

Suspensions of the p8^{+/+} or p8^{-/-} pBabe-ras^{V12}/E1A transformed MEFs or the p8^{+/+} or p8^{-/-}pBabe transduced MEFs (10⁶/200 µl PBS) were injected subcutaneously as xenografts into the flank of male athymic 7-8 week-old nu/nu mice, and tumours were allowed to develop for 20 days.

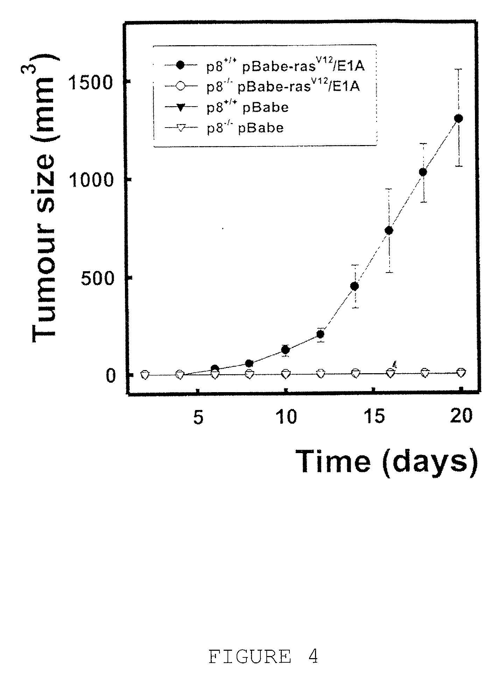

Tumours were measured externally with a caliper, in two dimensions, on the indicated days. Tumour volumes were determined from the equation: V=(L x W2) x 0.5, where L is length and W is width. The results are shown in Table I below and Figure 4. Tumourigenicity is defined as the number of mice with tumours/number of injected mice.

**Table I**

| Cells injected | Route of Inoculation | Tumourigenicity |
|---|---|---|
| p8^{+/+} pBabe MEFs | SC | 0/4 |
| p8^{-/-} pBabe MEFs | SC | 0/4 |
| p8^{+/+} pBabe-ras^{V12}/E1A MEFs | SC | 6/6 |
| p8^{-/-} pBabe-ras^{V12}/E1A MEFs | SC | 0/6 |

Similarly, transformed p8^{+/+} cells produced tumours in all athymic nude mice (6/6) when injected subcutaneously, whereas the transformed p8-deficient MEFs, like control cells, did not (0/6) (Table 1).

Figure 4 represents growth curves of p8^{+/+} and p8^{-/-} pBabe-ras^{V12}/E1A transformed MEFs or of p8^{+/+} and p8^{-/-} pBabe-transduced MEFs.

Legend of Figure 4:
―●― : p8⁺/⁺ pBabe-ras^{V12}/E1A
―○― : p8⁻/⁻ pBabe-ras^{V12}/E1A
―▼― : p8⁺/⁺ pBabe
―∇― : p8⁻/⁻ pBabe

Each data point represents the average tumour size of six mice for p8^{+/+} pBabe-ras^{V12}/E1A -transformed and p8^{-/-} MEFs or 3 mice for pBabe-transduced p8^{+/+} and p8^{-/-} MEFs. Error bars represent the SEM. Tumours that did not grow were assigned a volume of zero. Note that p8^{-/-} pBabe-ras^{V12}/ElAtransformed, as well as the control p8^{+/+} and p8^{+/+} pBabe-transduced MEFs did not develop tumours at all.

### In vivo spreading assays

The p8^{+/+} and p8-/-transformed MEFs were injected intraperitoneally to test their ability for dispersed growth. 10⁶ p8^{+/+} or p8^{-/-} pBabe-ras^{V12}/E1A-transformed MEFs or p8^{+/+} or p8^{-/-}pBabe-transduced MEFs cells in 200 µl PBS were injected into male nude mice and animals were killed after 20 days. Intraperitoneal injection leads to metastasis formation without the presence of a primary tumour.

The results are shown in Table II below. Spreading ability is defined as the number of mice with metastasis/number of injected mice.

**Table II**

| Cells injected | Route of Inoculation | Spreading ability |
|---|---|---|
| p8^{+/+} pBabe-ras^{V12}/E1A MEFs | IP | 6/6 |
| p8^{-/-} pBabe-ras^{V12}/E1A MEFs | IP | 0/6 |

Diffuse tumour growth tissue was observed into the abdominal cavity with pBabe-ras^{V12}/EIA transformed p8^{+/+} MEFs, but not in mice injected with Babe-ras^{V12}/EIA-transformed p8 deficient cells nor in p8^{+/+} or p8^{-/-} cells transduced with the control retrovirus, which were completely free of tumour formation 45 days post-injection.

### Expression of p8 mRNA in tumour cells.

p8 mRNA expression was estimated by RT-PCR in p8⁺/⁺ pBabe-ras^{V12}/E1A-transformed cells obtained from sub-confluent culture, in subcutaneous tumours and in intraperitoneal tumours.

RNA was extracted using the Trizol procedure and p8 and RL3 mRNAs expressions were analyzed as described in Example 2 above.

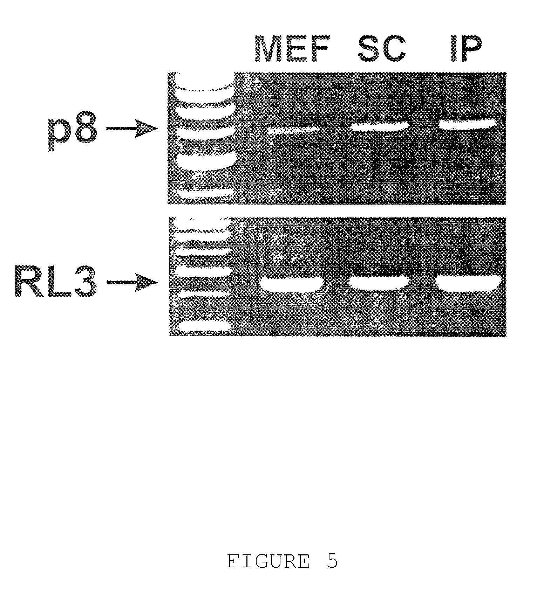

The results are shown in Figure 5. These results show the activation of p8 mRNA expression in subcutaneous (SC) and in intraperitoneal (IP) tumours, compared to p8⁺/⁺ pBabe-ras^{v12}/E1A transformed cells (MEF)

Therefore, although p8^{+/+} and p8^{-/-} MEFs seem to be similarly transformed by the pBabe-ras^{V12}/EIA retroviral vector as judged by their behaviour in standard culture conditions (cf. Example 2), the inability of the transformed p8-deficient cells to form colonies in soft-agar, to form subcutaneous tumours or generate intraperitoneal spreading, and the fact that in transformed p8^{+/+} MEFs, p8 mRNA expression is activated in subcutaneous as well as in intraperitoneal tumours, show that expression of p8 is required for the organization and development of tumours.

## Claims

1. The use of an inhibitor of the expression or of the activity of p8/com1 for preparing an antitumoral medicament.

2. The use of claim 1, wherein said inhibitor is selected among:
- triple-helix-forming molecules capable of sequence-specific binding with double-helical DNA in the p8/com1 promoter;
- antisense molecules specifically binding with p8/com1 mRNA;
- ribozymes directed to cleave the p8 mRNA.

3. An antitumoral medicament comprising an inhibitor of the expression or of the activity of p8/com1.


SEQUENCE LISTING

<110> INSERM

<120> USE OF INHIBITORS OF EXPRESSION OR ACTIVITY
      OF p8/com1 FOR TREATING TUMOURS

<130> MJPcb598-55

<140>
<141>

<160> 4

<170> PatentIn Ver. 2.1

<210> 1
<211> 21
<212> DNA
<213> Artificial Sequence

<220>
<223> Description of Artificial Sequence: PRIMER

<400> 1
ggagagagca gactaggcat a                                           21


<210> 2
<211> 21
<212> DNA
<213> Artificial Sequence

<220>
<223> Description of Artificial Sequence: PRIMER

<400> 2
gttgctgcca cccaagggca t                                           21


<210> 3
<211> 20
<212> DNA
<213> Artificial Sequence

<220>
<223> Description of Artificial Sequence: PRIMER

<400> 3
gaaagaagtc gtggaggctg                                             20


<210> 4
<211> 20
<212> DNA
<213> Artificial Sequence

<220>
<223> Description of Artificial Sequence: PRIMER

<400> 4
atctcatcct gcccaaacac                                                    20